# EUROPEAN PATENT APPLICATION

(11) **EP 4 374 826 A1**
(43) Date of publication of application: **29.05.2024**
(21) Application number: 22209133.2
(22) Date of filing: 23.11.2022
(51) Int. Cl.: A61F 2/24

(54) **A FIXATION DEVICE FOR IMPLANTATION**

(71) Applicant: The Provost, Fellows, Foundation Scholars, and The Other Members of Board, of The College of The Holy and Undivided Trinity of Queen Elizabeth, Dublin 2 (IE)
(72) Inventor: Murphy, Bruce, Dublin (IE); Hughes, Brian, Dublin (IE); Whelan, Alison, Dublin (IE)
(74) Representative: Tomkins & Co

(57) **Abstract**

A fixation device for fastening to a native heart valve having native valve leaflets, the fixation device comprising:
(a) an expandable body, the expandable body having: (i) a collapsed configuration with a minimum cross-sectional area; (ii) a fully expanded configuration with a maximum cross-sectional area; and (iii) one or more intermediate configurations where the, or each, intermediate configuration has a cross-sectional area that is intermediate that of the collapsed configuration and that of the fully expanded configuration;
(b) a locking mechanism for locking the expandable body in at least one intermediate configuration; and
(d) at least one clamp on the device body, the clamp for engaging and holding a native valve leaflet.

This device allows repositioning after implantation. Also disclosed is a fixation device including a pinion mechanism including a pinion wherein the pinion mechanism is configured for rotating the moveable jaw about the pivot axis. Also disclosed is a fixation device including a deployable shield that allows tissue to be caught by the clamp when the clamp is in a closed position but deploys to prevents tissue being caught by the clamp when the clamp is an least one open configuration.

## Description

### Field of the Invention

The present invention relates to a fixation device for implantation. For example it may be a fixation device for fastening to tissue such as a native heart valve having native valve leaflets. It may be configured as a stent or a valve. It may be configured as a stent with a valve mounted thereon/therein. It may be configured as a coaptation assist stent that is covered with a material. Of particular interest is a device of the invention in the form of a prosthetic valve, such as a prosthetic heart valve.

### Background to the Invention

The use of fixation devices within the body, for example within the vasculature, is well known. For example it is well known to provide replacement artificial valves in the heart when native valve(s), for example mitral or tricuspid valve, are dysfunctional. Artificial valves can repair or replace lost valve function.

Examples of such devices are described for example in patent publications US2011/137397 (Edwards Lifesciences); US2016/324633 (Mitraltech), EP3654886 (Murphy et al), US Patent No. 7,635,329 to Evalve Inc., and US Patent No. 10,905,552 (Edwards Lifesciences).

Notwithstanding devices provided in the past, it remains a challenge to provide a fixation device that can be implanted and secured in an optimal position. For example, during a procedure, optimal positioning may be difficult to achieve especially on a first attempt and re-positioning of the device one or more times may be required during an implantation procedure. So the device has to allow for re-positioning yet still adequately fix itself in place when a final position is selected. This is especially the case for devices implanted percutaneously.

In the case of a fixation device used to fix an artificial valve in place, the issue that arises is, that correct positioning of the device, to allow the artificial valve to repair or replace function of a native valve, is necessary. Accordingly for artificial valves the positioning of the fixation device and in particular a valve it carries is critical. Given that a main objective is to have the function of a native heart valve supplemented or replaced by the artificial valve positioning is especially key as bad positioning can mean the artificial heart valve allows undesirable blood flow when the valve is closed for example allowing regurgitation.

In the case of fixation devices described in US Patent No. 7,635,329 to Evalve Inc., and US Patent No. 10,905,552 (Edwards Lifesciences), correct positioning can be difficult. In some cases coaptation of the valve leaflets may not be achieved when the device has been attached onto the valve leaflets. If this occurs the device can be repositioned. However, in some cases clinically meaningful regurgitation still exists after the placement of a first device. In this case, the user may opt to implant a second clip to reduce the residual regurgitation. Furthermore, in some cases more than two devices may be implanted to reduce or eliminate regurgitation. This can be time consuming and can introduce additional risks during the procedure. It also requires a great degree of skill to eliminate regurgitation with one device or an array of multiple devices. There is no option to adapt these devices live during the procedure; either they must be repositioned or additional devices must be implanted to reduce regurgitation.

Notwithstanding all of the solutions proposed to date it remains an objective to provide an implant device that can be secured in place within the body but which allows for ease of repositioning if required. Also it is desirable to provide a device that will be retained securely in place. Another desire is to allow the user the ability to adapt the device to decrease regurgitation, when a single device has been attached to the valve leaflets. Another desire is to allow the device to be delivered with a minimal invasiveness. If a device is to be implanted with minimal invasiveness then the device suitably is capable of being delivered percutaneously and typically by transcatheter. It is particularly desirable to provide such a device that can remain in place despite being subject to natural movement of the body. For example a device used to repair and/or replace heart valve function must be capable of remaining in place despite the motion of a beating heart and associated blood flow.

The present invention addresses these challenges by allowing repositioning, providing an adaptable solution, yet locking securely in a finally selected position, and is capable of being delivered percutaneously and typically by transcatheter.

### Summary of the Invention

In one aspect, the present invention provides a fixation device for fastening to a native heart valve having native valve leaflets, the fixation device comprising:
(a) an expandable body, the expandable body having: (i) a collapsed configuration with a minimum cross-sectional area; (ii) a fully expanded configuration with a maximum cross-sectional area; and (iii) one or more intermediate configurations where the, or each, intermediate configuration has a cross-sectional area that is intermediate that of the collapsed configuration and that of the fully expanded configuration;
(b) a locking mechanism for locking the expandable body in at least one intermediate configuration; and
(c) at least one clamp on the device body, the clamp for engaging and holding a native valve leaflet.

The present invention relates to implant devices, for example implant devices for native valves within an animal body in particular a human body. Of particular interest are heart valves. The device of the invention may be configured as a coaptation assist stent. Optionally it may be provided with an external sheath or covering that acts as a conduit to confine blood flow to flow in an axial direction through the centre of the device. Optionally the conduit may contain a valve, the valve being configured to be delivered minimally invasively and act to inhibit blood flow from the ventricle to the atrium.

The implant device of the invention can be secured in place within the body but allows for ease of repositioning if required. It provides a device that will be retained securely in place. A device of the invention is configured to allow the user the ability to adapt the device to decrease regurgitation, for example when a single device has been attached to the valve leaflets. Another desire is to allow the device to be delivered with a minimal invasiveness.

A device of the invention can be implanted with minimal invasiveness. The device suitably is capable of being delivered percutaneously and typically by transcatheter techniques.

A device of the invention can remain in place despite being subject to natural movement of the body. For example a device used to repair and/or replace heart valve function must be capable of remaining in place despite the motion of a beating heart and associated blood flow.

The present invention addresses these challenges by allowing repositioning, yet locking securely in a finally selected position. A device of the invention is capable of being delivered percutaneously and typically by transcatheter. A device of the invention thus provides an adaptable solution

The device of the invention is for use with valves that are dysfunctional and in particular for use with valves which do not close properly or otherwise allow blood flow through the valve when closed, for example due to perforation. In other cases the valve may close but the annulus across which it closes is dysfunctional, for example it may be dilated. Dilation may cause malcoaptation. Such dysfunctional valves are often referred to as leaky/leaking valves. Valves that do not close properly impair the pumping function of the heart, as blood pumped by the heart can leak back through the valve instead of being pumped out through the vasculature. The present invention is particularly useful for dysfunctional mitral valves. While the present invention is described in relation to the mitral valve it will be appreciated that it is intended for application to other valves such as the tricuspid valves.

The intermediate configuration(s) may be an expanded configuration.

Desirably in a fixation device according to the invention at least one, and desirably all, intermediate configuration(s) include a change of shape of the expandable body.

A change of shape allows for more closely fitting to a native tissue shape, such as the annulus of a native valve. Additionally or alternatively a change of shape allows for closely matching the regurgitant area shape.

Desirably the locking mechanism has a plurality of locking positions so that the locking mechanism can lock the expandable body in more than one intermediate configuration. This allows for adaptability during implantation as the device shape can be altered after implantation within a patient's body.

In one configuration the expandable body has a resilient bias towards the collapsed configuration. Alternatively the expandable body has a resilient bias towards the fully expanded configuration. Alternatively the expandable body has a resilient bias towards an intermediate configuration.

The locking mechanism comprises one or more bands about the expandable body. The band(s) can be utilised to change the expandable body to a shape/size in such an intermediate configuration. Once a selected shape/size has been achieved the locking mechanism can be activated to hold the band(s) and thus the expandable body in that selected configuration. For example the locking mechanism can hold the band(s) against movement. Optionally the band(s) apply a tensile force to the expandable body. The bands hold the expandable body in its desired configuration. In this respect the bands may resist radial expansion and/or contraction of the expandable body.

Desirably at least one band, and suitably all bands, is/are inside the expandable body.

It is desirable that the locking mechanism comprises at least one bracing element that is configured to hold the expandable body in at least one intermediate configuration. The bracing element(s) may be in addition to or an alternative to the band(s). Optionally the bracing element(s) resists forces such as those causing a radial expansion and/or contraction of the expandable body.

A bracing element allows for good control of the shape and size of the expandable body when in an expanded configuration.

Optionally the bracing element is extendable, for example wherein the bracing element is twisted and at least partially untwists to move to an extended configuration.

Desirably the locking mechanism comprises a plurality of bracing elements that connect to the expandable body. The bracing elements can be sized and spaced about the expandable body to achieve a desired end shape/size. For example the bracing elements can be differently sized so that the elongate body expands further in one direction than another, for example in directions substantially perpendicular to each other such as to a more elongated elliptical shape.

In one arrangement the bracing element(s) have one end joined to the expandable body and a second end joined to a moveable carrier, wherein movement of the moveable carrier allows an intermediate configuration to be selected. This is a simple yet highly effective way of controlling the expandable body size/shape. It will be appreciated that the design of the bracing elements(s) can be such to impart a desired shape/size change to the device.

Desirably the moveable carrier can be locked against movement so as to hold the expandable body in an intermediate configuration. This means that one device can have many different configurations in shape/size and these can optionally be locked in to the device.

One simple yet highly effective arrangement is where the locking mechanism comprises a collet that can be opened or closed, preferably closed, to effect a locking action. For example it can be closed to lock band(s) or the position of the moveable carrier.

Desirably the expandable body takes the form of a stent. It will be collapsible for delivery and then expandable (one at a target site) for implantation.

Optionally the expandable body is formed from a nitinol material. It may have a default memory for a desired configuration, e.g. fully expanded, collapsed or intermediate.

Desirably the clamp comprises at least one moveable jaw, the moveable jaw having thereon at least one gripping element to grip a native valve leaflet.

Optionally the gripping element is moveable relative to the moveable jaw between a native valve leaflet gripping configuration of the moveable jaw where the gripping element is arranged to grip a native valve leaflet and a passive configuration of the moveable jaw where the gripping element is arranged so that it cannot grip a native valve leaflet.

The device of the invention is very adaptable. Not only can its final selected size for implantation be selected, but also its shape can be selected because the size and/or the shape of the expandable body can be changed during the procedure and then locked for final implantation. This means it can be delivered in its collapsed configuration and then expanded at the target site. Subsequently the size and shape can be adjusted to an intermediate configuration or the largest configuration. The configuration that is best suited in size and shape to match the target site is then chosen in the procedure and locked in position. And this size and shape can be selected during an implantation procedure.

The one or more intermediate configurations can have a different shape to either or both of the collapsed configuration and the fully expanded configuration as the expandable body. And any intermediate configuration can have a different shape to another. Either or both of the shape and the cross-sectional area of the expandable body can be different for different intermediate configurations.

For example the present invention can comprise an adaptable heart valve device that optimises: (1) the number of devices implanted, and (2) the clinical outcomes for the patient.

The device of the invention can be considered a fixation device that can change shape minimising leakage across a native valve annulus, such as occurs with MR (mitral regurgitation). For example severe MR can cause increased back flow of blood across the left ventricle which may result in increased left atrial and pulmonary artery pressure. This can lead to consequential issues for other regions of the heart. The device of the invention may also minimise the mean trans-valvular pressure gradient. By being able to alter a shape the best compromise between the residual MR and minimising any increase in mean pressure gradient can be found.

Furthermore, the function of allowing a change of shape and/or size should have the effect of reducing the number of implants used in a procedure for a given patient as the device of the invention can be adapted to a desired shape *in-situ* (within a patient) during a procedure. This should also have the effect of optimising the remaining residual regurgitation with one device.

In terms of desirable shape and/or cross-sectional area as is applicable to all embodiments, it is desirable to change shape towards the shape of the annulus of a native heart valve and this is a generally elliptical shape (including an elongated ellipse where the shape is generally elliptical but may for example be stretched in the direction of a major axis). For example a desirable change of shape may be to impart a changing force that changes the shape from an elliptical shape with a shorter major axis to an elliptical shape with a longer major axis. This includes a change to a larger cross-sectional area. And this shape can then be locked into the device by the locking mechanism. For example the device can be designed to be longer (e.g. having a major axis) in the commissure to commissure direction than in the anterior-posterior leaflet direction.

A device of the invention as described above can further comprise a pinion mechanism including a pinion, wherein the pinion mechanism is configured for rotating the moveable jaw about the pivot axis. This pinion mechanism is described below and herein.

In another aspect, which is combinable with all embodiments of the present invention, the present invention also relates to a fixation device for implantation in an animal, comprising:
(a) a device body;
(b) at least one clamp on the device body, the clamp for engaging and holding tissue so as to hold the fixation device in place, such as the tissue of a native valve leaflet, the clamp comprising at least one moveable jaw, the moveable jaw being rotatable about a pivot axis; and
(c) a pinion mechanism including a pinion, and wherein the pinion mechanism is configured for rotating the moveable jaw about the pivot axis.

This provides a simple yet highly effective method of moving one or more moveable jaws. It also allows for substantial force to be applied to open or close the moveable jaw in a simple and reliable manner. It also allows the jaw to be locked in place by locking the pinion mechanism. Furthermore it can accommodate differing degrees of closure for example due to more or less tissue being clamped by the moveably jaw. This allows for reliable capture of tissue. And of course if it is desired to release captured tissue, for example for the purposes of repositioning etc. then this mechanism works well to allow such release and capture.

Desirably the pinion is on the moveable jaw, for example, integrally formed with the moveable jaw. This allows for fewer parts and provides a moveable jaw that can take considerable force. Again this allows for release and recapture.

Desirably the pinion has an axis of rotation that is coincident with the pivot axis of the moveable jaw. This simplifies the construction and requires fewer parts and a pivoting force can be transmitted directly to a pinion on the arm. Again this allows for ease of release and recapture.

Optionally the pinion mechanism is a rack and pinion mechanism that includes a rack. Again a simple and robust construction that allows for ease of release and recapture.

Suitably the rack is moveable in a reciprocating linear fashion, so that linear motion of the rack effects pivotal movement of the moveable jaw about the pivot axis. Linear motion is easy to apply using a delivery mechanism and is easily controlled by a user.

Desirably the rack has teeth and the pinion has teeth and the two sets of teeth mesh with each other. The rack may have a rack body on which the teeth are formed and the teeth of the rack project above an upper surface of the rack body. Optionally the rack has a rack body in which the teeth are formed and the teeth of the rack are flush with an upper surface of the rack body.

The pinion mechanism may further comprises a worm screw and the worm screw and the pinion are configured so that rotation of the worm screw rotates the pinion to effect rotation of the moveable jaw about the pivot axis. This is an alternative to a rack and the worm screw can translate a rotational motion about its axis to impart an opening or closing action to the moveable jaw.

Optionally the pinion mechanism includes two pinions, on opposing sides of the moveable jaw.

A device of the invention as described above can have an expandable body, the expandable body having: (i) a collapsed configuration with a minimum cross-sectional area; (ii) a fully expanded configuration with a maximum cross-sectional area; and (iii) one or more intermediate configurations where the, or each, intermediate configuration has a cross-sectional area that is intermediate that of the collapsed configuration and that of the fully expanded configuration. Such as described above and herein.

A device of the invention as described above can additionally have a locking mechanism for locking the expandable body in at least one intermediate configuration. This locking mechanism is described below and herein.

It will be appreciated that the embodiments of the invention described as having a locking mechanism can be combined in any desired way with the embodiments of the invention including a pinion mechanism. Indeed in the detailed description and drawings embodiments with such combinations are described.

In another aspect, which is combinable with all embodiments of the present invention, the present invention also relates to a fixation device for implantation in an animal, comprising:
(a) a device body;
(b) at least one clamp on the device body, the clamp for engaging and holding tissue so as to hold the fixation device in place, such as the tissue of a native valve leaflet, the clamp comprising at least one moveable jaw, the moveable jaw being rotatable about a pivot axis; and
(c) a deployable shield that allows tissue to be caught by the clamp when the clamp is in a closed position but deploys to prevent tissue being caught by the clamp when the clamp is an least one open configuration.

As will be appreciated the deployable shield can prevent unwanted catching on tissue, for example during release and repositioning. This is especially important where the moveable jaw is configured for catching tissue.

Optionally the deployable shield is a flexible member that is pulled taut to form the deployable shield. This is a simple yet highly effective arrangement. For example then the clamp is open the shield may be deployed/become operable.

This aspect of the invention works well where the moveable jaw has one or more tissue gripping elements thereon and the deployable shield prevents tissue being gripped by the gripping element(s) in an at least one open configuration.

Desirably the moveable jaw can open to an inverted position and the deployable shield is deployed in the inverted position. Inversion is a configuration in which the moveable jaw(s) may be moved into a position suitable for withdrawal from a clamped position.

It will be appreciated that the embodiments of the invention described as having can be combined in any desired way with the embodiments of the invention including an expandable body, the expandable body having: (i) a collapsed configuration with a minimum cross-sectional area; (ii) a fully expanded configuration with a maximum cross-sectional area; and (iii) one or more intermediate configurations where the, or each, intermediate configuration has a cross-sectional area that is intermediate that of the collapsed configuration and that of the fully expanded configuration; and/or a locking mechanism and/or a pinion mechanism. All of the foregoing are described above and herein.

Indeed in the detailed description and drawing embodiments with such combinations are described.

### Brief Description of the Drawings

Embodiments of the invention will be described, by way of example only, with reference to the accompanying drawings. It will be appreciated that some figures are drawn to different scale for ease of illustration.
**Figure 1** shows a fixation device of the invention entering the left atrium via an introducer catheter;
**Figure 2** shows a perspective view of a fixation device of the invention in a collapsed configuration with a minimum cross-sectional area;
**Figure 3** shows a perspective view of the fixation device of **Figure 2** in an expanded configuration;
**Figures 4A to 4E** show a sequence that illustrates a locking mechanism of the device of **Figures 1 to 3****;**
**Figure 5** shows a perspective view of an alternative fixation device of the invention in a collapsed configuration;
**Figure 6** shows a perspective view of the fixation device of **Figure 5** in an expanded configuration;
**Figure 7** shows a perspective view of the fixation device of **Figures 5** and **6** showing in more detail al locking mechanism;
**Figure 8** shows a perspective view of an alternative fixation device of the invention in a collapsed configuration;
**Figure 9** shows a perspective view of the fixation device of **Figure 8** in an expanded configuration;
**Figure 10** shows a side view of the fixation device of **Figure 8** in an expanded configuration;
**Figures 11A** to **11C** show a partial detailed view of the fixation device of **Figures 8** to **10** showing in more detail a locking mechanism;
**Figure 12** shows a perspective view of an alternative fixation device of the invention in a collapsed configuration;
**Figure 13** shows a perspective view of the fixation device of **Figure 12** in an expanded configuration;
**Figure 14** shows a perspective view of an alternative fixation device of the invention in an expanded configuration;
**Figure 15** shows a perspective view of the fixation device of **Figure 14** in an expanded configuration and with the moveable jaws in an inverted configuration;
**Figure 16** shows one or more optional implant positions of a device of the invention in the heart;
**Figure 17** shows the device in **Figure 16** but with the delivery controls removed.
**Figure 18** shows an alternative device of the invention on a delivery device; and
**Figures 19A** to **19C** show a sequence of positions of the moveable arms of the device of **Figure 18****.**

### Detailed Description of the Drawings

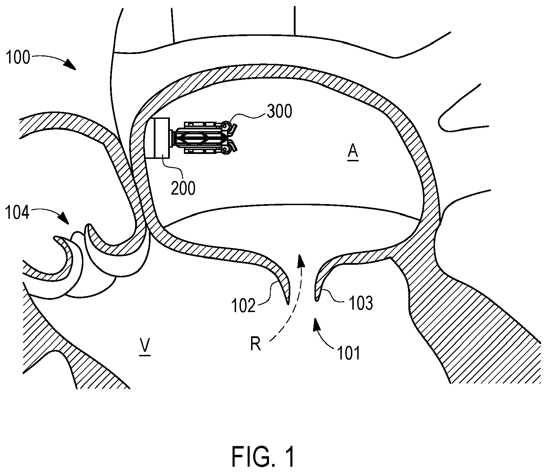

**Figure 1** shows an enlarged sectional view of a part of a heart 100. The heart 100 is illustrated in the systolic phase, just prior to the aortic valve 104 opening. In this phase the mitral valve 101 should be sealed closed, with no gap between its two leaflets 102,103. However there is a gap between the two leaflets 102,103, allowing blood to flow backwards into the left atrium **A.** This blood flow is indicated by dashed arrow **R.** In this particular case there is functional mitral valve disease causing regurgitation through the mitral valve 101. As explained above this reduces heart function. Heart failure may result because of this leaky mitral valve.

Also illustrated in **Figure 1** is a distal end of an introducer catheter 200 within the left atrium **A,** the introducer catheter is protruding through the atrial septal wall. The placement of this introducer catheter 200 has been carried out via a precursor step of a transseptal puncture. (Similar to the step that would be conducted as part of a procedure for a left atrial appendage transcatheter procedure.) This introducer catheter 200 acts as a delivery conduit to deliver guide catheters or implant delivery systems transcutaneously. In this position the introducer catheter 200 can be used to deliver interventional tools, implantation devices or catheters into the left atrium/ventricle.

A fixation device 300 of the invention is also shown and will be described in more detail below. It has been advanced through the introducer catheter 200 until it has appeared in the atrium **A.** It is for delivery to the mitral valve 101 as will be described below.

There are a number of options available for steering an implant such as a fixation device 300 of the invention and any suitable option may be used. A first method includes using an introducer catheter to gain access to the left atrium (as per **Figure 1****).** Subsequently a guide catheter that is steerable is introduced through the introducer. The guide catheter is then steered into the correct position and a separate implant delivery system is delivered through the steerable guide catheter and pushed out into the correct position in a target site, for example, in the heart.

In a second method a steerable introducer catheter along with a steerable guide catheter can be used to place the fixation system in the correct position.

In a third method an introducer catheter is used to gain access to the left atrium (as per **Figure 1****).** In this case there is no steerable guide catheter. Instead the implant delivery system is steerable and can be used to deliver the device to the correct position and orientation within the heart.

This latter method is used in the figures.

The fixation device 300 is show in in a compact configuration for travel through the introducer catheter 200/vasculature. The configurations of device 300 will be described in more detail below.

It is to be noted that the fixation device 300 would typically be provided with an external sheath or covering but it is not shown to allow for ease of depiction of a fixation device of the invention. For example it may have a material such as a biocompatible material covering its external parts. The covering could be made of a material such as: PTFE including ePTFE (expanded PTFE), pericardium, PET, polypropylene, Nylon, polyurethane, silicone, polyethylene including UHMWPE (ultrahigh molecular weight polyethylene). The covering's porosity can be varied to encourage fibrous tissue ingrowth or additionally it could be altered to cause certain components to be less permeable and have less or no tissue ingrowth. E.g. the stent element 3100 component could have a covering that is less permeable than the covering that encases the outside surface of the moveable jaws. Preferably, the material that covers the moveable jaws would be suitable to enhance fibrous tissue ingrowth. There are many combinations of coverings that can be used on a clinically implantable device. Different areas could have a cover with different properties.

The attachment of the covering to the fixation device 300 of the invention could be achieved by: tying (for example using sutures), adhesive, welding, fasteners (such as micro rivets), spraying or weaving (for example by weaving a cover onto the fixation device). Different portions of a fixation device of the invention could have covers with different properties and/or be manufactured and/or applied with different processes.

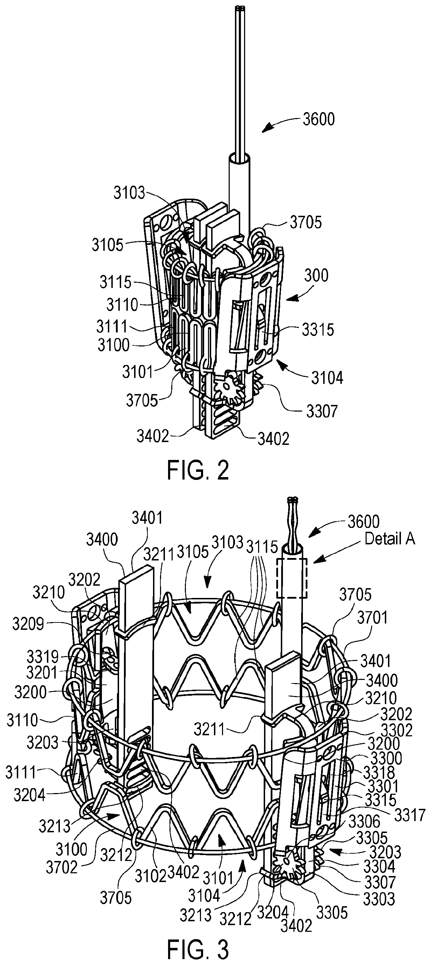

**Figure 2** shows a perspective view of a fixation device 300 of the present invention. The fixation device 300 is for fastening to a native heart valve having native valve leaflets. It comprises a central spacer, core element or stent element 3100. This stent element forms the expandable body of the fixation device.

This stent element 3100 is formed by an open frame 3101 formed from interconnecting scaffolding parts 3102. The frame 3101 forms a base frame on which other parts of the fixation device 300 are mounted. The frame 3101 has an open top end 3103 and an open bottom end 3104 generally defining a central lumen 3105. The stent element 3100 thus defines a central space or conduit which runs through the centre of frame 3101.

It will be appreciated that a valve can be carried within the frame 3101 and/or the frame can be completely covered with a non-permeable covering material. The valve within the central lumen 3105 thus imparts a valve functionality to the fixation device 300. And the covering material, covering the sides and top and bottom of the frame 3101 does not allow blood to flow through the central lumen 3105 thus forms a blocking spacer that blocks blood flow. This will have the effect of filling the regurgitant area between the heart valve leaflets and creating a seal to minimise regurgitation.

The frame 3101 comprises two generally aligned but spaced apart and generally concentric respective upper and lower ring elements 3110 and 3111 which are generally configured to be the same in construction. The upper and lower ring elements 3110 and 3111 are each formed by a series of arms 3115. The arms 3115 are generally v-shaped and in the embodiment the v-shape of the upper ring element 3110 is a mirror image of that of the v-shape of the lower ring element 311.

Overall this arrangement forms a stent element 3100 which is generally cylindrical. This cylindrical shape may be in an as-cut shape. Furthermore the stent element 3100 is deformable. For example the arms 3115 are desirably deformable. For example they may be stretched (e.g. to a more open V-shape) to expand the cross-sectional area of the stent element 300 as illustrated for example in **Figure 3****.**

In particular the stent element 3100 is an expandable body which has a collapsed configuration with a minimum cross-sectional area (see for example the configuration of **Figure 2****)** and an expanded configuration with a larger cross-sectional area (see for example the configuration of **Figure 3****).** It will be appreciated that the stent element 3100 has a plurality of intermediate configurations where each intermediate configuration has a cross-sectional area that is intermediate that of the collapsed configuration and that of the fully expanded configuration.

The stent element 3100 can be formed of any suitable material. For example it may be cut from a suitable material, for example, cut from a tube of suitable material. For example it may be laser cut. It may for example be made from nitinol, titanium, stainless steel or cobalt chrome.

As is desirable for all embodiments the fixation device 300 further comprises a locking mechanism for locking the expandable body formed by the stent element 3100 in at least one intermediate configuration.

As is desirable in all embodiments, the locking mechanism of this embodiment allows, not only for the stent element to be locked in an intermediate configuration, but also for a change of shape to be imparted to the stent element 3100. This is achieved by locking different parts of the stent element 3100 at different positions (where those parts have different cross-sectional areas and/or shapes) if desired.

In this embodiment the locking mechanism includes respective bands, with band 3701 on the upper ring element 3110 and band 3702 on the lower ring element 3111. The bands 3701 and 3702 extend about the expandable body formed by the stent element 3100.The bands 3701 and 3702 are each attached to the respective upper ring element 3110 and lower ring element 3111 by a series of slide rings 3705. The band 3701 and the upper ring element 3110 extend through one series of side rings and the band 3702 and the lower ring element 3111 extend through the other series of slide rings 3705.

It will be appreciated then that by enlarging or contracting the bands 3701 and 3702 the cross-sectional area of the stent element 3100 can be selected.

Additionally or alternatively, by enlarging or contracting the bands 3701 and 3702, the shape of the stent element 3100 can be selected.

Additionally or alternatively by enlarging and/or contracting the bands 3701 and 3702 to a differing extent the shape of different parts of the stent element 3100 can be selected.

In other words a change in shape and/or cross-sectional area can be applied differentially across the stent element 3100 to impart a non-uniform change in shape and/or cross-sectional area to the stent element 3100. For example one band could be used to enlarge and the other used to contract etc. In this case the stent element 3100 can be constructed of Nitinol.

For example the bands 3701 and 3702 could be used to apply a tensile force to contract an expanded stent element 3100. And as above the tensile force can be of a different magnitude to impart different shape and/or cross-sectional area. Optionally the band(s) apply a tensile force to the expandable body. The bands hold the expandable body in its desired configuration. In this respect the bands may resist radial expansion and/or contraction of the expandable body.

In terms of desirable shape and/or cross-sectional area as is applicable to all embodiments, it is desirable to change shape towards the shape of the annulus of a native heart valve and this is a generally elliptical shape. For example a desirable change of shape may be to impart a changing force that changes the shape from an elliptical shape with a shorter major axis to an elliptical shape with a longer major axis. This includes a change to a larger cross-sectional area. And this shape can then be locked into the device by the locking mechanism.

An important aspect of all devices of the present invention is that these changes to shape and/or cross-sectional area can be imparted when the device is inside a patient's body. So over the wire changes are possible. This means that during a procedure the shape and/or cross-sectional area of a device can be adapted to best suit the shape and/or cross-sectional area of the annulus of a native valve of a heart.

In the embodiments the device 300 comprises two opposing non-moving jaws 3200 which form part of a clamp of the fixation device 300 of the invention. Each non-moving jaw 3200 is formed by an elongate arm 3201 which has a proximal end 3202 and a distal end 3203. A transverse bore 3204 is defined in the non-moving jaw 3200. The transverse bore 3204 allows for pivotal connection of a moving jaw as will be described in more detail below.

Respective non-moving jaws 3200 are held onto opposing sides of the stent element 3100 by any suitable joining mechanism including welding fastening etc. It is to be noted that they are positioned inside the stent element 3100. The non-moving jaws 3200 can also form part of the stent element 3100 and for example may be integrated into the stent element as it is formed for example by cutting. For example they may be formed by being cut into a substrate such as a tube or sheet from which the stent element is formed. Cutting can be achieved by laser cutting. The laser cut pattern of the base tube or sheet.

There is an aperture 3209 defined in each of arms 3201. These apertures 3209 accommodate respective gripping elements in a native valve leaflet gripping configuration of the moveable jaw. They also accommodate gripped tissue. More detail is given below. It will be appreciated that each moveable jaw can have a plurality of gripping elements. There can be as many apertures 3209 as there are gripping elements so that each aperture functions with a respective gripping element as a grip to hold tissue.

The stent element 3100 also acts as a support or holder for a rack element as will be described below.

In particular the proximal end 3202 of each arm 3201 has formed thereon a bracket 3210 which terminates in a generally rectangular-shaped open-centred guide 3211 though which a rack element passes and in which the rack element is slidingly engageable. The distal end 3203 of each arm 3201 also has formed thereon a similar bracket 3212 which also terminates in a generally rectangular-shaped open-centred guide 3213 through which the rack element also passes and, again in which the rack element is slidingly engageable. These brackets could also be located at any intermediate point on each arm 3201. The brackets can be welded or joined to the arm. Alternatively they can be integrated into the stent element 3100 for example integrally designed into a laser cut pattern of the stent element 3100. More detail is given below.

Also part of a fixation device 300 of the invention are moveable jaws 3300. A respective moveable jaw 3300 together with its corresponding non-moving jaw 3200 forms a clamp of the fixation device 300 of the invention.

Each moveable jaw 3300 is formed by an elongate arm 3301 which has a proximal end 3302 and a distal end 3303. On each distal end 3303 is a hinge portion 3304. A pinion is provided in the form of a pair of pinion gears 3305. The pinion gears 3305 are on the moveable jaw, and may be integrally formed with the moveable jaw. The pinion gears 3305 effect movement of the moveable jaws 3300. The moveable jaw can be machined form a flat sheet and formed into its desired configuration. The moveable jaw can be injection moulded, or machined. It can be appreciated that the moveable jaw illustrated is manufactured by laser cutting a flat sheet and forming the jaw into its final configuration. If the jaw was machined or injection moulded the geometry could be optimised in a different manner.

A shaft, axle or pin 3306 passes through the transverse bore 3204 defined in the non-moving jaw 3200 and also through the hinge portion 3304 and the pinion gears 3305 making each moveable jaw 3300 pivotable relative to its corresponding non-moving jaw 3200. The respective pinion gears have an axis of rotation that is coincident with the pivot axis of the corresponding moveable jaw 3300. The pinion gears 3305 form part of a pinion mechanism.

On the moveable jaw 3300 a gripping element 3315 is provided in the form of resiliently deformable hooks or grips. A respective base 3317 of the two gripping elements 3315 is integrally formed with a respective arm 3301 within an aperture 3318 defined in the arm 3301. It can be appreciated that the configuration of the gripping element(s) can be formed in many different ways for example where the component has been formed from a sheet of material or a tube of material. Other configurations would be available if the component was machined or injection moulded or cast.

A top (tissue gripping) part 3319 of the gripping element 3315 stands proud of the moveable jaw 3300. This is a native valve leaflet gripping configuration of the moveable jaw 3300. The gripping element 3315 is resiliently deformable and is thus moveable relative to the moveable jaw 3300.

The gripping elements 3315 are arranged for gripping tissue. However the gripping elements 3315 may be retracted by a suitable force that causes them to deform e.g. flatten. Once the force is removed they will return (under their own resilient bias) to the configuration shown in **Figures 2 and 3****.**

As shown in **Figures 2 and 3** the respective gripping elements 3315, protrude through the respective apertures 3209 in the non-moving arms 3200. This means tissue can be gripped and held between the moving and non-moving arms as tissue will be forced into the apertures 3209 and/or against the periphery of the apertures 3209 by the respective gripping elements 3315. As mentioned above the gripping elements 3315 can deform so this allows for the closure of the device to its native valve gripping configuration with the tissue held.

As mentioned above each arm 3201 also acts as a support or holder for a rack element 3400. The rack element 3400 is a sliding element which forms part of a mechanism for opening and closing the moveable jaw 3300. The sliding element 3400 slides on and relative to the non-moving jaw 3200.

In particular the rack element 3400 forms part of the pinion mechanism. The rack element 3400 together with the pinion gears 3305 form a pinion mechanism. The rack element 3400 comprises a planar body 3401. The planar body 3401 is dimensioned to be accommodated within and carried by the non-moving arms 3200 and in particular the brackets 3210, 3212. In particular the planar body 3401 is dimensioned to be accommodated within and slidingly carried by the generally rectangular-shaped open-centred guides 3211, 3213 through which the rack element 3400 passes.

The planar body 3401 of the rack element 3400 also has defined therein a series of rack teeth 3402. These rack teeth 3402 mesh with pinion teeth 3307 on a pinion gear 3305. In particular each pair of pinion gears 3305 mesh with the pinion teeth 3307 on the rack element 3400, thus forming a rack and pinion mechanism for moving each moveable jaw 3300.

As the rack element 3400 is moveable in a reciprocating linear fashion, that linear motion of the rack element 3400 effects pivotal movement of the moveable jaw 3300 about the pivot axis defined by the shaft, axle or pin 3306.

Movement of the respective rack elements 3400 is independent of each other and so too are the moveable jaws 3300 which open and close independently of each other. A control mechanism or other such means can be used (for example through introducer catheter 200) to move the respective rack elements 3400. So again the extent of opening and closing of the moveable jaws 3300 is controlled from the exterior of the patient's body. Again this allows for implantation and repositioning if desired. If desired the rack elements can be controlled simultaneously rather than individually.

The moveable and non-moveable arms and indeed the rack element and pinion mechanism can be machined from a block of material, for example by traditional machining methods, or moulded. For example such components can be machined by EDM (electrical discharge machining). Such components can also be 3D printed if required. They can be made of any suitable material including metals and alloys such as stainless steel, cobalt, chrome, titanium, nitinol, or a polymer.

Furthermore such components could be manufactured from a tube or flat sheet and manipulated into the required shape (for example shape set NiTi or plastically deformed material) that encompasses all the features that are required in order for it to function.

And as is evident not only is the movement of the moveable arms 3300 independent of each other it will be appreciated that the shape and/or cross-sectional area of the device is independently selectable of the movement of the moveable arms 3300.

This means for example closing of one of the moveable arms to grip tissue can be carried out to position the device 300 and then its shape and/or cross-sectional area can be adjusted if desired before the second moveable arm is closed to grip tissue. And indeed any sequence of anchoring using closing of one or more of the arms and re-sizing and/or reshaping can be carried out. This gives maximum flexibility for initial positioning and then repositioning.

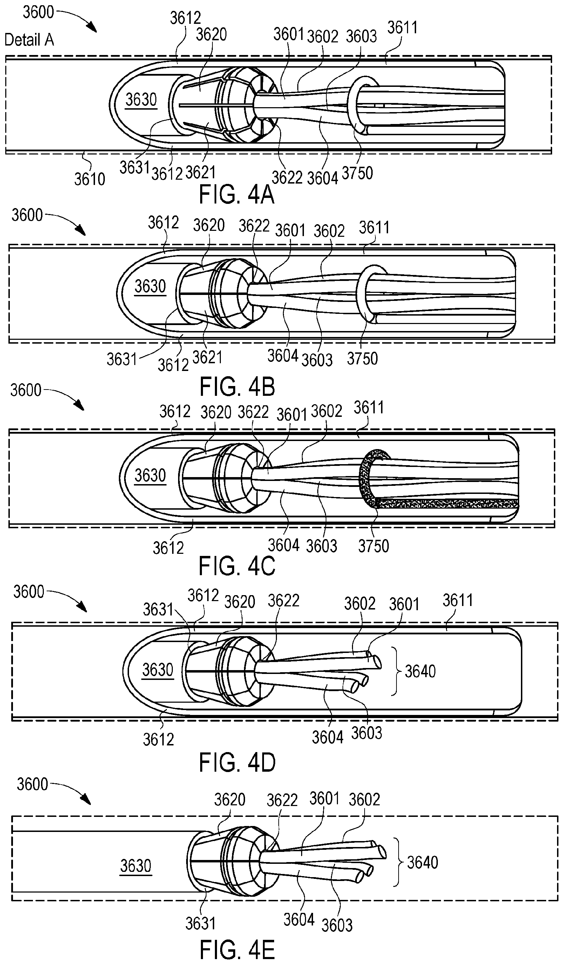

A schematic representation of a control mechanism 3600 is shown in the drawings and the "Detail A" view indicated in **Figure 3** is shown in **Figure 4****.** In particular **Figures 4A to 4E** show a sequence that is illustrative of a locking mechanism of the invention.

**Figures 4A to 4D** show a cut-out window 3612 (which is present only for the purposes of showing the internal workings) in a delivery tube 3610. Through that window the internal workings can be seen but in use no such window is present.

As mentioned above there is a band 3701 on the upper ring element 3110 and a band 3702 on the lower ring element 3111 and the bands 3701 and 3702 extend about the expandable body formed by the stent element 3100.

In this arrangement the band 3701 is formed by a loop which is continuous with elongate elements 3601 and 3602. It will be appreciated then by applying a compressive and/or tensile force through one or both of the elongate elements 3601 and 3602, the shape and/or cross-sectional area of the band 3701 can be adjusted. It will be appreciated that the forces applied can not only be different in quantum but also different in direction. Accordingly selectivity in shape and/or cross-sectional area is provided.

Similarly the band 3702 is formed by a loop which is continuous with elongate elements 3603 and 3604. Again by applying a compressive and/or tensile force through one or both of the elongate elements 3603 and 3604, the shape and/or cross-sectional area of the band 3702 can be adjusted. Again the forces applied can not only be different in quantum but also different in direction.

Optionally the band(s) apply a tensile force to the expandable body. The bands hold the expandable body in its desired configuration. In this respect the bands may resist radial expansion and/or contraction of the expandable body.

Furthermore it will be appreciated that the shape and/or cross-sectional area of the bands 3701 and 3702 can be different so the shape and/or cross-sectional area of the device 300 can be varied at different positions.

Once a desired shape and/or cross-sectional area has been selected this can then be locked so that there is no further change due to forces experienced by the device.

In this respect the locking mechanism comprises a collet 3620 formed by a series of segments 3621 and which has an opening 3622 about which the segments 3622 are arranged in a ring. The collet 3620 can be advanced out of or retracted into the mouth 3631 of a tube 3630. The elongate elements 3601-3604 all pass through collet 3620 and in particular mouth 3622 thereof. The advancing or retraction action of the collet 3620 allow the collet 3620 to release or grip the elongate elements 3601-3604.

The sequence is shown in **Figures 4A** to **4E****.**

In **Figure 4A** the collet 3620 is shown advanced from tube 3630 and the mouth 2622 thereof is open. This allows for adjustment of the bands 3701 and/or 3702 (and thus the device 300) to any extent that is desired as movement of the elongate elements 3601-3604 is not restricted as longitudinal movement through the collet 3620 is allowed.

Once the desired shape and size configuration of the device 300 is selected the collet 3620 is retracted into the mouth 3631 of the tube 3630 to close the segments 3622 toward each other and thus close the mouth 3622 about the elongate elements 3601-3604. This provides a locking action/mechanism. In this case the bands 3701, 3702 are now locked against movement and so the configuration of the device 300 is locked. Such a locked configuration is shown in **Figure 4B****.**

In the drawings there is shown a heating or cauterising loop or coil 3750, such as an induction coil or the like. In **Figures 4A** and **4B** the heating loop 3750 is positioned about the elongate elements 3601 to 3604. In this configuration and while the adjustments described above are taking place the heating loop 3750 is inactive and does not interfere with the movement of the elongate elements 3601 to 3604.

Once the configuration of the device 300 has been selected as in **Figure 4B** then the sequence from **Figure 4C** to **4E** can be followed. As indicated by the shading in **Figure 4C** the heating loop 3750 is activated to heat. This causes the severing of the elongate elements 3601 to 3604 so that they have truncated ends 3640 as shown in **Figure 4D****.** The heating loop 3750 has been removed in **Figure 4D****.** Furthermore in **Figure 4E** the tube 3610 has also been removed.

This means the device 300 can be left in its implanted position with all connections to a control device removed.

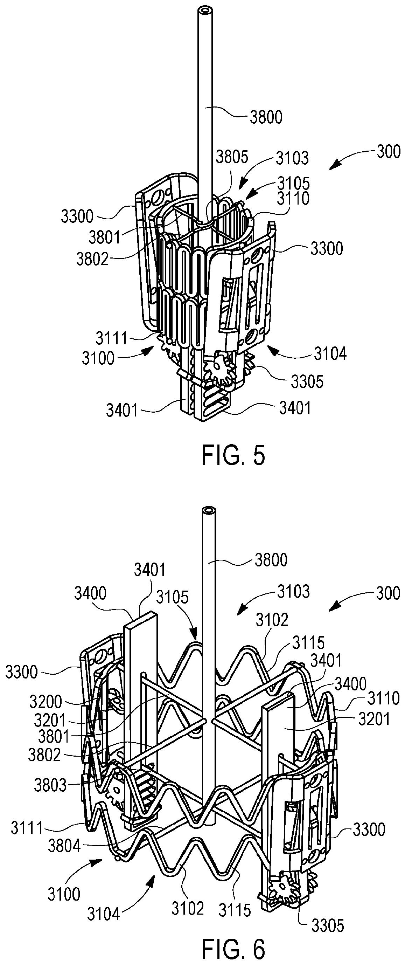

**Figures 5** and **6** show a device which is very similar to that of earlier drawings. In this respect the same parts are present. The device 300 is again for fastening to a native heart valve having native valve leaflets. It comprises a central spacer, core element or stent element 3100. This stent element forms the expandable body of the fixation device. A covering (has not been shown), comprising of a material such as ePTFE or PET or other suitable material, covers the sides of the stent element 3100 and also covers the bottom and the top of the stent element 3100.

This stent element 3100 is formed by an open frame 3101 formed from interconnecting scaffolding parts 3102. Again the stent element 3100 is an expandable body which has a collapsed configuration with a minimum cross-sectional area (see for example the configuration of **Figure 5****)** and an expanded configuration with a larger cross-sectional area (see for example the configuration of **Figure 6****).** It will be appreciated that again the stent element 3100 has a plurality of intermediate configurations where each intermediate configuration has a cross-sectional area that is intermediate that of the collapsed configuration and that of the fully expanded configuration.

Also the non-moving jaws 3200 (each formed by an arm 3201) and the moveable jaws 3300 work in the same manner as described above. Rack elements 3400 work in the same manner as described above. The rack element 3400 is a sliding element which forms part of a mechanism for opening and closing the moveable jaw 3300. The sliding element 3400 slides on and relative to the non-moving jaw 3200.

Again the rack element 3400 forms part of the pinion mechanism. The rack element 3400 together with the pinion gears 3305 forms a pinion mechanism.

Other than the differences mentioned below the embodiment of **Figures 5** and **6** works as described above.

An alternative locking mechanism is utilised in the embodiment of **Figures 5** and **6****.** In this embodiment the locking mechanism comprises at least one bracing element that is configured to hold the expandable body in at least one intermediate configuration.

The bracing element applies a force to hold the stent element 3100 in a certain configuration. For example where the stent element is configured to be biased towards a collapsed configuration the bracing element, which may take the form of a strut, resists the compressive force of the stent element and holds it open. For example where the stent element is configured to be biased towards an expanded configuration the bracing element resists the outward radial force, which may be in a circumferential or radial direction, of the stent element and holds it in that position against that tensile force. In this respect it will be noted that when the stent element is configured to be biased towards an expanded configuration the bracing element is not required to have any compressive strength and may thus be flexible and for example act as a tie line. Additionally or alternatively the strut(s) may flex.

In **Figures 5** and **6** the stent element 3100 is biased toward an expanded configuration. In this configuration the stent element may be constructed of Nitinol for example shape set to its largest configuration. The stent element is thus configured to be biased towards an expanded configuration. In this embodiment the bracing element, which may take the form of a tensile member for example a tensile tie line or member, resists the expansive force of the stent.

The locking mechanism comprises a central shaft 3800 that extends into the central lumen 3105. As is desirable for all embodiments, there are four bracing elements 3801 to 3804.

All four bracing elements 3801-3804 extend through the central shaft 3800 and join to opposing sides of the stent element 3100. Bracing elements 3801 and 3802 are arranged substantially at right angles to each other (within the same transverse plane of the device) and extend through the central shaft 3800 and between opposing sides of the upper ring element 3110. Bracing elements 3803 and 3804 are arranged substantially at right angles to each other (within the same transverse plane of the device) and extend through the central shaft 3800 and between opposing sides of the lower ring element 3111.

Opposing rotational movement of the central shaft 3800 can be used to lengthen or shorten the bracing elements 3801 to 3804. In particular as shown in **Figure 5** which is a collapsed configuration of the fixation device 300 the bracing elements 3801 to 3804 have been shortened by twisting or winding about the central shaft 3800. (In the view of **Figure 5** only the bracing elements 3801-3802 can be seen forming a winding 3805 - the bracing elements 3803-3804 also form a like winding but they cannot be seen in this view.) In this arrangement the bracing elements are twisted or wound and at least partially untwist or unwound to move to an extended configuration.-

This means that the bracing elements 3801 to 3804 are extendable (and retractable) by a desired amount which is controlled by the amount (and direction) of rotation of the central shaft 3800. In **Figure 6** the bracing elements 3801 to 3804 are fully extended having been fully released for example by being unwound from the central shaft 3800.

In order not to interfere with the movement of the respective rack elements 3400 slots 3403 have been provided in the respective rack elements 3400 to accommodate movement relative to the bracing elements 3801 and 3803.

It will be appreciated that the bracing elements 3801 to 3804 may have differing lengths to each other to impart a desired shape change.

Again it will be appreciated that the shape and/or cross-sectional area of the device can be altered during a surgical procedure while the device is being implanted.

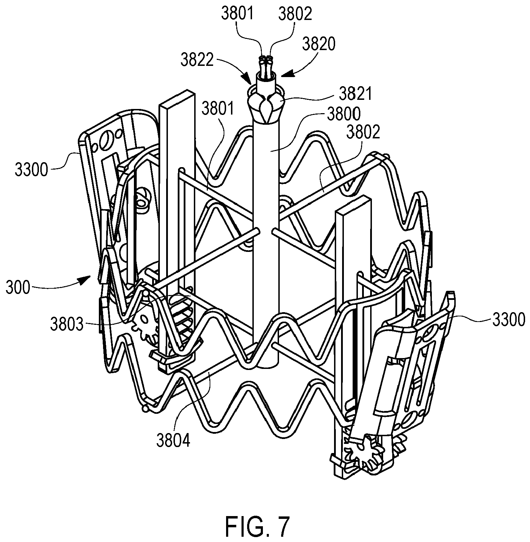

**Figure 7** shows a device of the type shown in **Figures 5** and **6****.** However in this particular configuration the bracing elements 3801 and 3802 are pulled vertically into and up the tube 3800 and locked using a collet mechanism in a manner similar to that described earlier. The locking mechanism comprises a collet 3820 formed by a series of segments 3821 and which has an opening 3822 about which the segments 3822 are arranged in a ring. The bracing elements 3801, 3802 extend through the opening 3822.

The collet 3820 can be closed to lock against the tension in the bracing elements 3801, 3802.

If desired the stent frame can be modified to include additional fixation points for the distal and proximal parts of the tube 3800 to hold it in a set position.

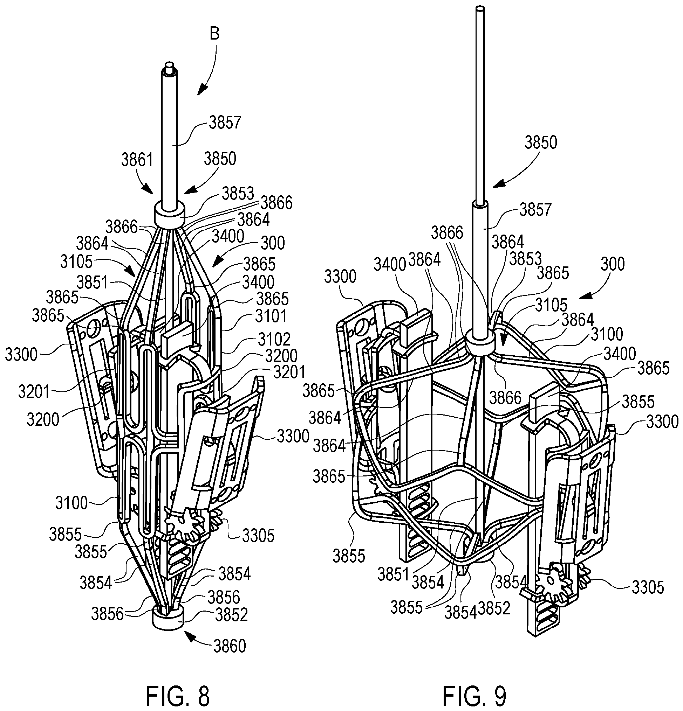

**Figures 8** and **9** show a device which is very similar to that of earlier drawings in particular the device of **Figures 2** to **4****.** In this respect the same parts are present. The device 300 is again for fastening to a native heart valve having native valve leaflets. It comprises a central spacer, core element or stent element 3100. This stent element forms the expandable body of the fixation device. The covering element which covers the sides, top and bottom of the stent element 3100 has not been illustrated for clarity of the underlying components.

This stent element 3100 is formed by an open frame 3101 formed from interconnecting scaffolding parts 3102. Again the stent element 3100 is an expandable body which has a collapsed configuration with a minimum cross-sectional area (see for example the configuration of **Figure 8****)** and an expanded configuration with a larger cross-sectional area (see for example the configuration of **Figure 9****).** It will be appreciated that again the stent element 3100 has a plurality of intermediate configurations where each intermediate configuration has a cross-sectional area that is intermediate that of the collapsed configuration and that of the fully expanded configuration.

If the stent element 3100 is constructed of nitinol, the shape set configuration could be the small configuration or the large configuration or any intermediate position. This would act as the resting configuration of the stent element 3100. Displacement of the proximal and distal ends of the stent element 3100 away from each other would decrease the overall cross sectional area, while displacement of the proximal end of the stent element 3100 towards the distal end of the stent element 3100 would cause the cross section of the stent element 3100 to increase.

Also the non-moving jaws 3200 (each formed by an arm 3201) and the moveable jaws 3300 work in the same manner as described above. A rack element 3400 is also included. The rack element 3400 is a sliding element which forms part of a mechanism for opening and closing the moveable jaw 3300. The sliding element 3400 slides on and relative to the non-moving jaw 3200.

Again the rack element 3400 forms part of the pinion mechanism. The rack element 3400 together with the pinion gears 3305 form a pinion mechanism.

Other than the differences mentioned below the embodiment of **Figures 8** and 9 works as described above in relation to opening and closing of the moveable jaws 3300.

An alternative locking mechanism is utilised in the embodiment of **Figures 8** and **9****.** The locking mechanism does not include bands such as those described above in relation to **Figures 2** to **4****.** In this embodiment (in common with **Figures 5** to **7****)** the locking mechanism comprises at least one bracing element that is configured to hold the expandable body in at least one intermediate configuration. As in all embodiments the bracing elements can form part of the stent element 3100 or can be separate elements that can be attached to the stent element. More particularly it comprises a plurality of bracing elements.

The bracing element applies a force to hold the stent element 3100 open. In this case the stent element may be configured to be biased towards a collapsed configuration. But whether or not it is, the bracing element, takes the form of a series of struts which are adapted to resist any compressive force (or indeed tensile force) acting on the stent element. Optionally the bracing element(s) resist radial expansion and/or contraction of the expandable body.

For example if the desired intermediate configuration (in which the device is to be locked) is smaller than the resting configuration of the stent element 3100 the bracing element(s) may apply a tensile force to the stent element 3100. So the bracing element(s) may assist in holding the stent element 310 to a desired shape. For example in a configuration that has been shape set in a resting position that is the largest configuration or an intermediate configuration the bracing element will pull the stent element into a smaller configuration. Irrespective of the forces applied to the stent element 3100 the series of struts hold it in a desired (selected) configuration.

In the embodiment the locking mechanism 3850 further comprises a central rod 3851.The central rod 3851 is located centrally within the central lumen 3105. At a distal end 3860 of the central rod 3851 there is a hub, joiner or coupler 3852. In the embodiment the coupler 3852 is fixed to the central rod 3851.

At a more proximal position 3861 of the central rod 3851 there is a moveable hub or carrier 3853. The moveable carrier is moveable along the central rod 3851. The moveable carrier is attached to a moveable sleeve 3857. The moveable carrier can act as a runner along the central rod 3851.

At the distal end of the stent element 3100 there are a series of struts 3854. A first end 3855 of each of the struts 3854 is attached to the stent element 3100. The struts 3854, in particular the respective first ends 3855, are thus attached to the stent element in a spaced apart arrangement and disposed about the periphery of the stent element 3100.

A second end 3856 of each of the struts 3854 is attached to the coupler 3852. The struts 3854, in particular the respective second ends 3856, are thus attached to the coupler 3852 in a spaced apart arrangement and disposed about the periphery of the coupler 3852.

At the proximal end of the stent element 3100 there are a further series of struts 3864. A first end 3865 of each of the struts 3864 is attached to the stent element 3100. The struts 3864, in particular the respective first ends 3865, are thus attached to the stent element in a spaced apart arrangement and disposed about the periphery of the stent element 3100.

A second end 3866 of each of the struts 3864 is attached to the moveable carrier 3853. The struts 3864, in particular the respective second ends 3866, are thus attached to the moveable carrier 3853 in a spaced apart arrangement and disposed about the periphery of the moveable carrier 3853.

As is desirable for all embodiments struts 3854/3864 are positioned on opposing sides of the stent element 3100.

As is desirable for all embodiments the struts are not attached to the stent element 3100 at a position in which they would interfere with a closed/gripping position of the moveable jaws 3300. The struts are disposed on the stent element at positions about the periphery of the stent element away from the moveable jaws 3300 (when the moveable jaws are in the closed positions).

In particular it is to be noted that the struts on the respective sides of the stent element are disposed equidistant to each other about the periphery of the stent element at positions, on the respective sides, about the periphery of the stent element that are equidistant about the periphery of the stent element away from the moveable jaws 3300 (when the moveable jaws are in the closed positions). For example where there are components comprising struts and moveable jaws the components are desirably located (such as being centred) equidistantly apart about the periphery of the stent element. For example in the embodiment where there are four struts and two moveable jaws there are 6 components. These components are desirably located (centred) approximately 60 degrees apart about the periphery of the stent element.

It is desirable that the upper and lower struts are arranged in the same way.

As in all embodiments struts around the circumference of the stent element can be arranged to impart different levels of expansion or contraction in different directions. For example, the struts could be designed with bend regions in the areas that oppose the heart valve leaflets and be straight elements in the areas of the device that face the commissures of the heart valve. This would have the effect that as the device expands, the shape can become substantial longer in the commissarial direction than in the opposite direction. Whereby the bend regions would bend and not apply an outward force after a pre-set value of expansion, while the straight strut regions will continue to apply an outward expansion to the stent element, resulting in a stent frame that is not axisymmetric in shape.

As in all embodiments it is desirable that the moveable jaws are located (diametrically) opposite to each other (centred) approximately 180 degrees apart about the periphery of the stent element.

As mentioned a desirable shape and/or cross-sectional area that is applicable to all embodiments, is to change shape towards the shape of the annulus of a native heart valve and this is a generally elliptical shape. For example a desirable change of shape may be to impart a changing force that changes the shape of the stent element from an elliptical shape with a shorter major axis to an elliptical shape with a longer major axis. This includes a change to a larger cross-sectional area. And this shape can then be locked into the device by the locking mechanism.

In this respect, and as applies to all embodiments of the invention, it is desirable that the moveable jaws 3300 are centred on an axis of the device that is the minor axis. Accordingly the moveable jaws are best positioned for gripping a leaflet and/or annulus of a native valve. For example the moveable jaws (and optionally a minor axis) may be centred for positioning in the anterior-posterior leaflet direction. And the major axis is optionally in the commissure to commissure direction

It is to be noted that the struts are of equal length. This means force applied (as described in more detail below) will be equally applied.

However in a device of the invention where greater elongation is desired in a direction, suitably the major axis direction, for example in a commissure to commissure direction, the struts can be configured to cause preferential elongation in that desired direction. For example the strut length in the commissure to commissure direction could be longer than the strut length in a than in the anterior-posterior leaflet direction.

For example the positioning of the struts in the present embodiment ensures that the stent element 3100 expands towards a more elliptical shape by imparting a greater change in dimensions along a major axis (and the moveable jaws 3300 are centred on the minor axis).

It is desirable that a device of the invention is symmetrical about (an increased) major axis.

In the embodiment the struts 3854/3864 are of equal length. This means force applied by each strut (as described in more detail below) will be approximately equal.

Furthermore the stent element is adapted so that it does not expand symmetrically. As will be appreciated from the foregoing and the drawings, the stent element is adapted to expand along the major axis. This may for example be achieved by having the stent element less deformable at positions (e.g. along a minor axis) proximate the position of the moveable arms 3300.

It is to be noted that, as is desirable for all embodiments, each of the series of struts from a generally conically-shaped arrangement. As is desirable for all embodiments, the struts together with the stent element, form a substantially cylindrical shape capped at its ends with a conical shape. When expanded, as described above, the cylindrical and conical shape(s) each expand to a greater extent along a major axis so that the cross-sectional shape of the conical shape(s) and the cylinder shape become progressively more elliptical, for example along a major axis.

In particular as a user imparts a force in the direction of **arrow B** this moves the moveable carrier 3853 in the direction of **arrow B** causing the struts 3853, 3854 to move. In particular the force in the direction of **arrow B** causes an increasing compressive force to be imparted between the coupler 3852 (which is fixed to the central rod 3851) and the moveable carrier 3853. This causes a progression from the configuration shown in **Figure 8** to that shown in **Figure 9****.**

As is desirable for all embodiments the stent element 3100 and the struts 3853, 3854 flex to allow such movement. For example they may be resiliently deformable. In any case such movement may take place within the elastic limits of the stent element 3100.

It will again be appreciated that the device can be changed to any intermediate configuration that is desired and such changes can be made after the device has been introduced into the body of a patient.

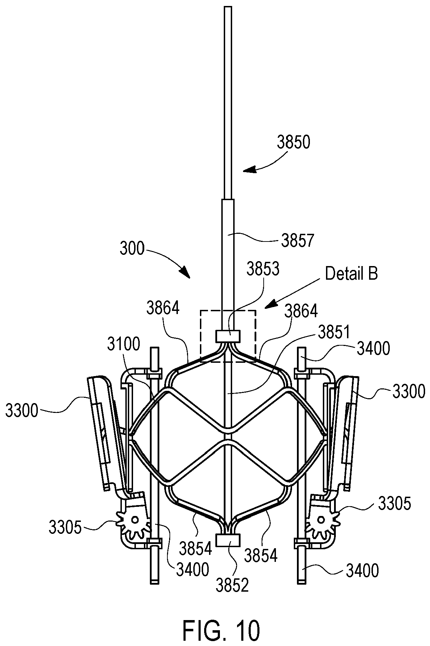

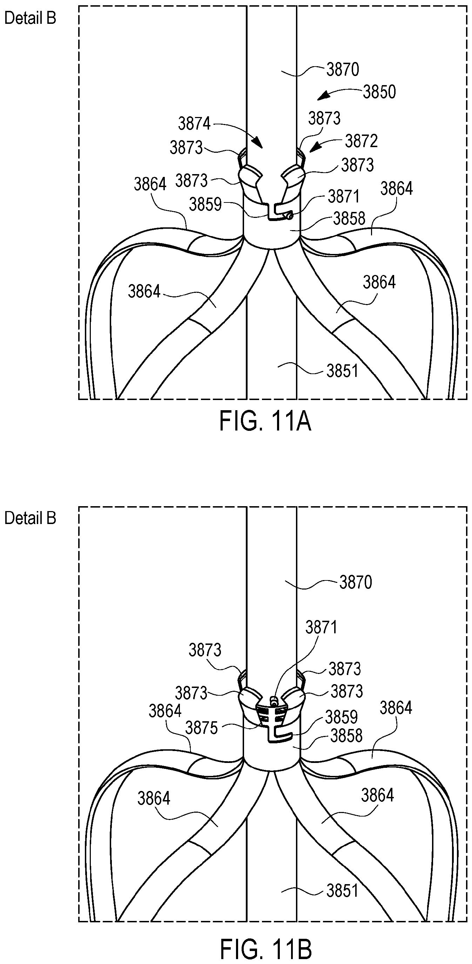

**Figure 10** shows a side view of the device 300 (which is in the same expanded state as shown in **Figure 9****).** **Figures 11A** to **11C** show further components of the locking mechanism which are internal to the portion of the device identified as "Detail B" in **Figure 10****.**

In particular at the top end of the struts 3864 is a collar or cuff 3858 within which is defined (on opposing sides thereof) an L-shaped recess 3859. A pin 3858 extending through a control rod 3870 can be utilised to engage the control rod 3870 with the cuff 3858 by inserting the pin into the recess. Once engaged the control rod 3879 can be used to impart the force mentioned above. The control rod 3879 engaged in this manner is shown in **Figure 11A****.**

Also present at the top end of the struts 3864 is a collet 3872 which is formed by segments 3873. The segments 3873 are arranged about a mouth 3874 through which the control rod 3870 extends.

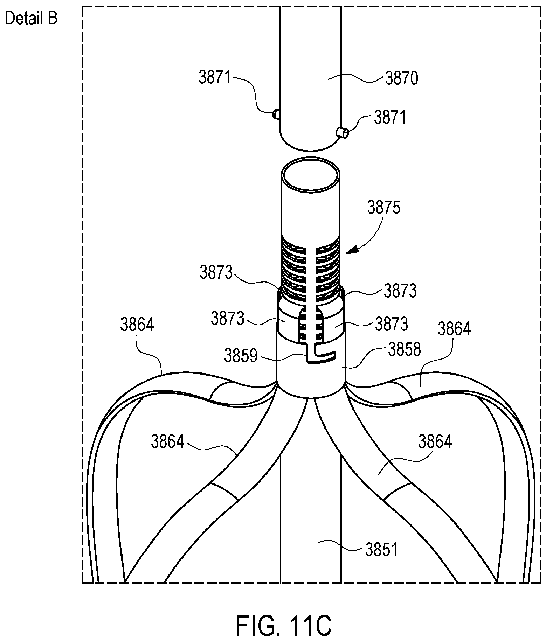

Once the device 300 is in a desired configuration the control rod 3870 can be removed by twisting the control rod 3870 to free the pin 3858 from the recess 3859 as shown in **Figure 11B****.** The control rod 3870 can then be completely disengaged as shown in **Figure 11C****.**

In particular as best seen in **Figures 11B** and **11C** defined in a proximal end of the central rod 3851 are a series of slots or apertures 3875. The collet 3872 and in particular the segments thereof can be closed about the central rod 3851 inserting the segments 3851 into respective ones of the slots 3875. In this way any further change to device 300 is locked out as the struts 3864 are locked against movement relative to the central rod 3851.

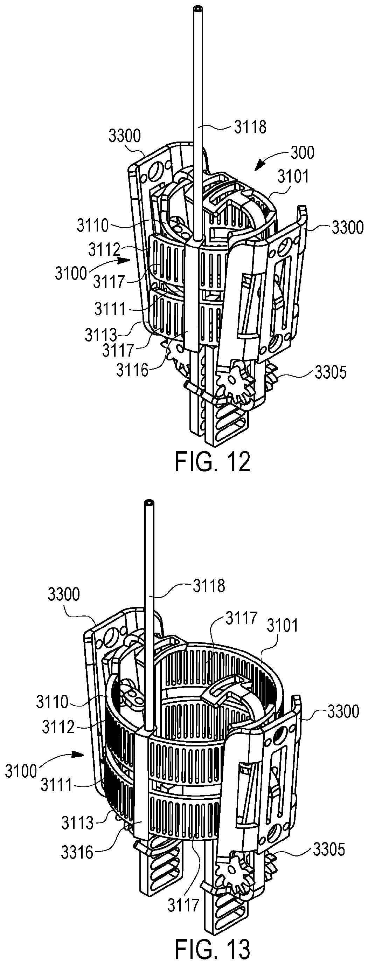

A further embodiment of a device of the present invention is shown in **Figures 12** and **13****.** Again the embodiment is similar to earlier embodiments save that a different locking mechanism is provided. Like earlier embodiments the frame 3101 of the stent element 3100 comprises two generally aligned but spaced apart and generally concentric respective upper and lower ring elements 3110 and 3111 which are generally configured to be the same in construction. However in contrast with earlier embodiments the upper and lower ring elements 3110 and 3111 are not each formed by a series of arms 3115. Instead they are each formed by a respective band 3112 and 3113.

The bands 3112 and 3113 pass through a rectangular retainer 3116 which and each have a degree of overlap as, at least in the configuration of **Figure 12** the bands have an excess length that can be utilised for expansion of the device. Each band and its overlap pass though the rectangular retainer 3116.

Each band 3112 and 3113 has a series of parallel apertures or slots 3117 defined therein.

Within the rectangular retainer is a mechanism, such as a worm gear or pinion gear which meshes with the parallel apertures or slots 3117 to allow adjustment of the size of the bands 3112 and 3113. Movement of the worm gear or pinion gear may be effected through a control rod 3118. Rotation of the worm gear or pinion gear in opposing directions causes the band(s) to increase or decrease in size. Furthermore the worm gear or pinion gear provides a locking mechanism preventing movement of the bands other than when the worm gear or pinion gear are moved by the user.

It will be appreciated that the two bands 3112, 3113 could be expanded or contracted in unison, for example using a common worm gear or pinion gear, but desirably, they are independently expanded or contracted for example by being independently operable by worm gear and/or pinion gear.

**Figure 13** shows the same device 300 as **Figure 12** but having been extended by operation of the mechanism. In **Figure 12** the bands 3112, 3113 have been expanded to the same amount, but as mentioned above, an alternative would be to expand them to differing amounts.

It will be appreciated that all embodiments of the invention have an adjustment mechanism to allow adjustment of the expandable body/stent element to one or more intermediate configurations. Furthermore once in an intermediate configuration the locking mechanism is utilised to lock the device in the desired configuration. However, as described immediately above, in some embodiments, the adjustment mechanism may also provide the locking functionality.

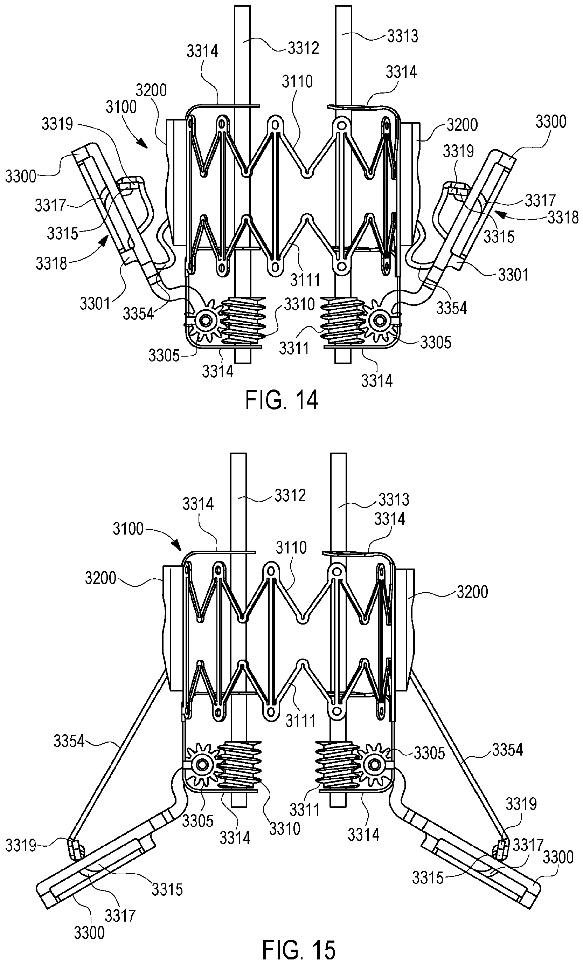

A further embodiment of a device of the present invention is shown in **Figures 14** and **15****.** Again the embodiment is similar to earlier embodiments. A locking mechanism similar to **Figures 2** and **3** can be provided but is not shown for ease of illustration of other components.

Like earlier embodiments the frame 3101 of the stent element 3100 comprises two generally aligned but spaced apart and generally concentric respective upper and lower ring elements 3110 and 3111 which are generally configured to be the same in construction. The device 300 has two opposing non-moving jaws 3200 which form part of a clamp of the fixation device 300 of the invention. It also has the moveable jaws 3300. Again the moveable jaws 3300 are moveable by rotation as described earlier using a pinion mechanism including a pinion gear 3305, on the moveable arms 3300.

One of the main differences between the embodiment depicted and those described earlier is the use of worm gears to effect movement of the moveable arms (instead of a rack element). As can be seen from **Figures 14** and **15** respective worm gears 3310 and 3311 are meshed with the respective pinion gears 3305 on the moveable arms 3300. Movement of the worm gears 3310 and 3311 are effected by control rods 3312 and 3313. It will be appreciated that rotational movement of the worm gears 3310 and 3311 can be used to effect opening and closing of the moveable jaws 3300.

In **Figure 14** the moveable arms 3300 are partially open having being moved away to a small extent from the respective non-moveable jaws 3200.

In **Figure 15** the moveable arms 3300 are fully open having being moved away from the respective non-moveably jaws 3200.

As described for earlier embodiments, on the moveable jaw 3300 is a gripping element 3315 is provided in the form of resiliently deformable hooks or grips. A respective base 3317 of the two gripping elements 3315 is integrally formed with a respective arm 3301 within an aperture 3318 defined in the arm 3301.

A top (tissue gripping) part 3319 of the gripping element 3315 stands proud of the moveable jaw 3300. This is a native valve leaflet gripping configuration of the moveable jaw 3300. The gripping element 3315 is resiliently deformable and is thus moveable relative to the moveable jaw 3300.

The gripping elements 3315 are arranged for gripping tissue. However the gripping elements 3315 may be retracted by a suitable force that causes them to deform e.g. flatten.

One important aspect of this inverted position is with both moveable jaws 3300 in an inverted position. By inverted position the inventors mean a position where a moveable jaw 3300 is in a position where a longitudinal axis of the moveable jaws 3300 form an angle of from about 110 to 190 degrees with a central longitudinal axis of the stent element 3100. This refers to the angle on the proximal side of the intersection of those longitudinal axes. Such a position is shown in **Figure 15****.** One important concern of this inverted position is that the gripping elements 3315 could catch in tissue such as chordae or the valve leaflets when the device is moved, for example during removal/re positioning.

This allows an opportunity to withdraw the device through the native valve and also possibly reinsert it into the ventricle.

For example when the device is pulled back into the left atrium **A** where the device can be manipulated to the closed configuration and/or it can pulled back into the delivery catheter. For example it may be configured to be withdrawn into the delivery catheter in this inverted position in particular where both moveable jaws 3300 are in the inverted position.

A respective tie line 3354 runs between the expandable body and the moveable jaws 3300, more particularly to the grips 3315. As the moveable jaws open (say for example the configuration of **Figure 14****)** the tie line remains slack, however as the moveable jaws move to an inverted position (say for example the configuration of **Figure** 15) the tie line becomes taut. It will be appreciated that as the tie line 3354 becomes taut it forms a shield or barrier that prevents tissue that comes into contact with the device 300 being caught by the moveable arm 3300/the grip 3315.

Thus, as is desirable for all embodiments, the present invention comprises a shield which deploys as a barrier which prevent gripping element(s) from catching on tissue.

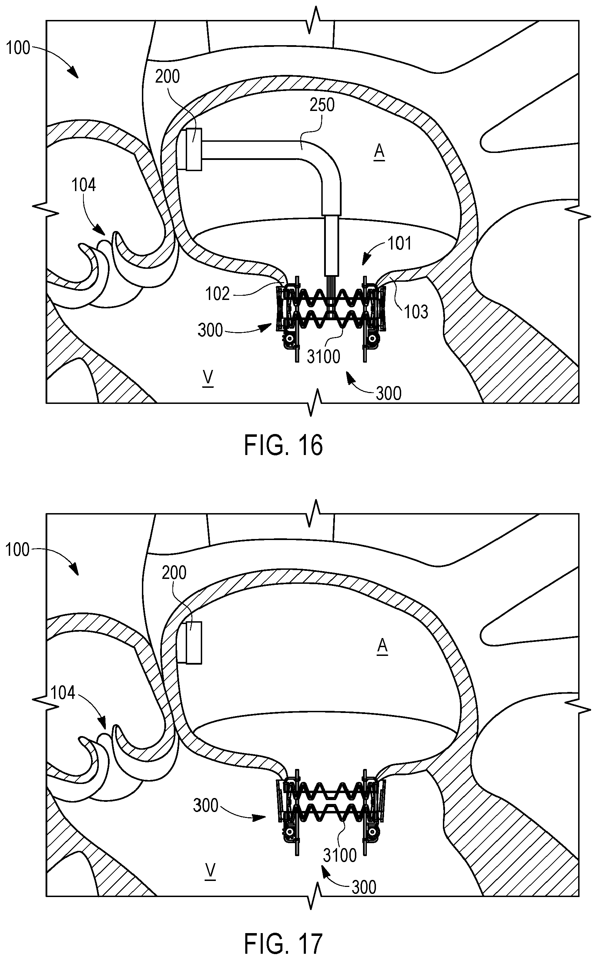

**Figures 16** to **17** show one or more optional implant positions in the heart 100 (without the device covering material). In **Figure 16** the implant delivery catheter 250 has been advanced through the introducer catheter 200. The implant delivery system/catheter's configuration has been manipulated or has a pre-set shape to allow it to approach the approximate plane of the mitral valve 101 in a perpendicular manner. The catheter's steering can be achieved by any suitable manner including utilising pull wires, pre-set mandrels or hydraulic actuation of a length of the catheter tubing close to the tip. This section of the catheter may be made up of a multicomponent system (such as a goose-neck system) or a flexible tube that deforms when tension is applied via pull wires or deforms when other mechanical action is applied or deforms when hydraulic pressures are altered in chambers of the catheter's walls.

In **Figure 16** the fixation device 300 is depicted in capture position where respective clips or clamps of the device close when a moveable jaw 3300 is closed against the corresponding non-moveable arm 3200. This ensures that an appropriate amount of tissue is grasped by the device. In particular this position may be achieved by first advancing the device 300 through the mitral valve 101 into the ventricle V and them moving it back and capturing the leaflets 102, 103.

The fixation device 300 is desirably orientated with the line of the moveable jaws 3300 being perpendicular to the line of coaptation of the anterior and posterior leaflet 102, 103. If the device was used in the tricuspid position, the device clip-line would be orientated perpendicular to the line of coaptation of the two target leaflets. The device's orientation, relative to the line of coaptation, is completed in the atrium A of the heart prior to advancing the implant into the ventricle V of the heart. The device will normally be advanced into the ventricle above the point that is causing the most regurgitation, or the point that the clinical team believes is the best position to deploy the device to eliminate regurgitation as part of a multi-clip deployment strategy.

As mentioned above the stent element 3100 may be expandable from a configuration with a lower cross-section profile to one which a larger cross-section profile. In **Figure 16** the stent element 3100 has been expanded to a desired shape/cross-sectional area as described above and locked in that configuration. As mentioned above it is possible for the person undertaking the procedure to change the shape and/or cross-sectional area while the procedure is taking place with the device within the heart of a patient. Furthermore the moveable jaws 3300 can be independently opened and closed multiple times if desired to capture and recapture a native leaflet for better positioning.

The device of the invention may be assembled together with a valve for example to form of a prosthetic valve, such as a prosthetic heart valve. For example a device of the invention may be implanted and then an artificial valve may be attached to it. So it may form a base structure to which an artificial valve may be attached. It thus forms an assembly comprising a device of the invention and an artificial valve.

For example a functioning transcatheter valve may be delivered. Once the device of the invention has been attached to the valve leaflets, it could be entered from the superior side with a guide wire and subsequently entered with a valve delivery system.

The artificial valve could be balloon expanded within the stent element and the stent element could act as a foundation structure for the delivered valve. For example if the stent element was 3 mm in diameter it would need to be pre-dilated to 19 mm to 30 mm to accept a transcatheter heart valve of the variety that are available to fix aortic stenosis. The placement of a transcatheter valve in the stent element 3100 could occur during the initial procedure or it could occur after months or years post the initial procedure.

This gives patients a potential further option subsequent to an initial mitral valve repair procedure.

In order for a valve to remain in a desired position within the fixation device 300, an interference fit between the stent element's 3100 structural frame 3101 and a frame of the transcatheter valve will result once the composite structure of the stent element 3100 and transcatheter valve have been combined.

In **Figure 16** both of the moveable jaws 3300 have been closed and the two heart valve leaflets 102,103 have been successfully captured. The moveable jaws 3300 are independently operable so they can be opened (simultaneously or individually) and the device can be repositioned, removed or a single moveable jaw 3300 can be opened and re-closed to try and achieve a better clinical outcome. In this configuration the heart valve leaflet tissue has been pushed through the apertures in the non-movable jaw by the gripping elements of the movable jaw. This configuration of gripping the tissue allows a robust durable solution for this long term implant.

**Figure 17** shows the device of **Figure 16** as implanted having being released from its delivery mechanisms.

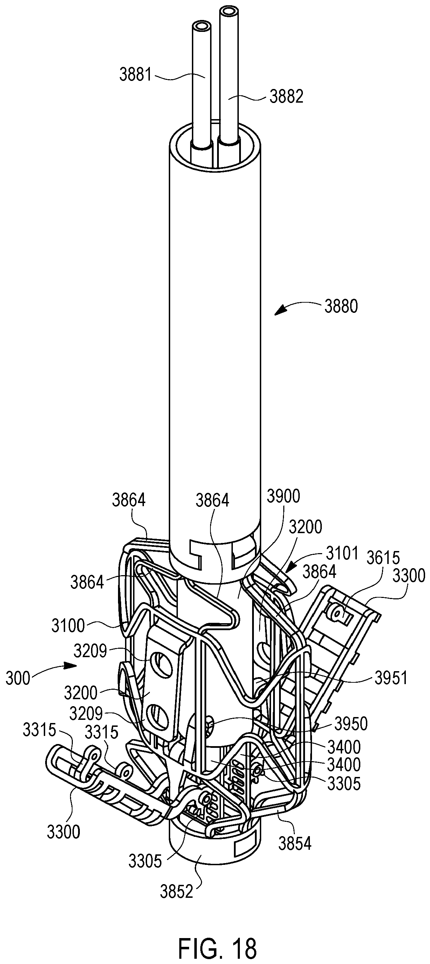

**Figure 18** shows an alternative device 300 of the invention on a delivery device. The device 300 is similar in construction to that shown in **Figures 8** to **11****.**

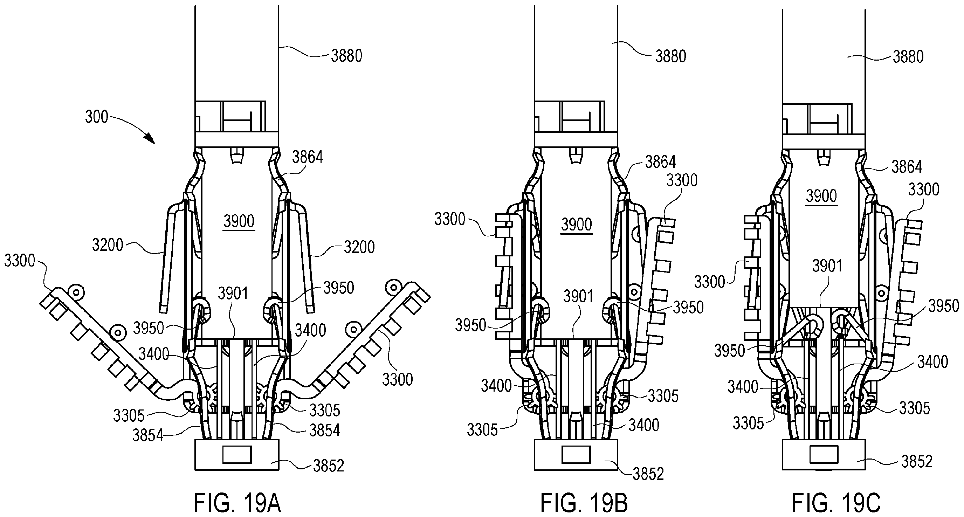

**Figures 19A** to **19C** shows a sequence of positions of the moveable arms of the device of **Figure 18****.**

Again respective rack elements 3400 form a sliding element which together with respective pinion gears 3305 form a pinion mechanism for opening and closing the respective moveable jaws 3300. Like other embodiments the moveable jaws 3300 together with non-moveable jaws 3200 form a clamp for gripping and holding native tissue. Like earlier embodiments at the distal end of the stent element 3100 there are a series of struts 3854. The struts 3854 are attached to the stent element in a spaced apart arrangement and disposed about the periphery of the stent element 3100. At the proximal end of the stent element 3100 there are a further series of struts 3864. The struts 3864 are thus attached to the stent element in a spaced apart arrangement and disposed about the periphery of the stent element 3100. Each of the struts 3854 is attached to a coupler 3852 as described previously. Each of the struts 3864 is attached to a moveable carrier 3853 which is not visible in **Figures 18** and **19****.** So the device 300 expands as described for the device shown in **Figures 8** to **11****.** Gripping elements 3315 are also provided on the moveable jaws 3300. As before these can push tissue into apertures 3209 in the non-moveable jaw 3200.

The main difference between the device 300 of this embodiment and other embodiments is the presence of a locking mechanism which in this arrangement is for locking the rack and pinion mechanism (against movement) and in turn locking the position of the moveable jaws 3300.

In this embodiment the locking mechanism comprises two resiliently deformable locking pawls 3950. In the embodiment these pawls 3950 form part of the stent element 3100 but of course may be independent of the stent element 3100. In the embodiment a deflection tube 3900 holds the pawls 3950 in a deflected position as shown in **Figure 18** and **Figures 19A** and **19B.**

**Figure 19A** shows the device 300 in a configuration with the moveable jaws 3300 in an open position. In **Figure 19B** the moveable jaws 3300 have been closed to capture tissue and it will be appreciated that as shown in **Figures 19B** and **19C** the moveable jaws 3300 can be closed to differing extents depending on the amount of tissue captured. In particular the moveable jaw 3300 to the right of the figures is not closed as much as that on the left, reflecting the amount of tissue captured.

In the sequence from **Figure 19B** to **19C** the deflection tube 3900 has been pulled upwardly so that a distal end 1901 of the deflection tube 3900 is withdrawn past the pawls 3950. The pawls 3950 spring inwardly and each catches a respective rack element 3400 as shown in **Figure 19C****.** It is to be noted that this locks the rack elements 3400 and in turn the moveable arms 3300 in their respective positions. When the pawls 3950 engage with the rack elements 3400 proximal movement of the rack element is restricted.

Movement of the struts and/or the rack elements and/or the deflection tube can be controlled through a delivery control mechanism within a delivery tube 3880 and for example with further control tubes 3881 and 3882.

As mentioned above it is to be noted that the fixation device 300 would typically be provided with an external sheath or covering but it is not shown to allow for ease of depiction of a fixation device of the invention. Also the artificial valve that would be carried (intemally) by the stent element 3100 is not shown to allow for ease of depiction of a fixation device of the invention.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A fixation device for fastening to a native heart valve having native valve leaflets, the fixation device comprising:
(a) an expandable body, the expandable body having: (i) a collapsed configuration with a minimum cross-sectional area; (ii) a fully expanded configuration with a maximum cross-sectional area; and (iii) one or more intermediate configurations where the, or each, intermediate configuration has a cross-sectional area that is intermediate that of the collapsed configuration and that of the fully expanded configuration;
(b) a locking mechanism for locking the expandable body in at least one intermediate configuration; and
(c) at least one clamp on the device body, the clamp for engaging and holding a native valve leaflet.

2. A fixation device according to any preceding claim wherein at least one, and desirably all, intermediate configuration(s) include a change of shape of the expandable body.

3. A fixation device according to any preceding claim wherein the locking mechanism has a plurality of locking positions so that the locking mechanism can lock the expandable body in more than one intermediate configuration; and/or wherein the expandable body has a resilient bias towards the collapsed configuration.

4. A fixation device according to any preceding claim wherein the locking mechanism comprises one or more bands about the expandable body, optionally wherein the bands apply a tensile force to the expandable body and further optionally wherein the at least one band is inside the expandable body.

5. A fixation device according to any preceding claim wherein the locking mechanism comprises at least one bracing element that is configured to hold the expandable body in at least one intermediate configuration; optionally wherein the bracing element resists radial expansion and/or contraction of the expandable body.

6. A fixation device according to Claim 5 wherein the bracing element is extendable, for example wherein the bracing element is twisted and at least partially untwists to move to an extended configuration.

7. A fixation device according to any of Claims 5 to 6 wherein the locking mechanism comprises a plurality of bracing elements that connect to the expandable body.

8. A fixation device according to any of Claims 5 to 7 wherein the bracing element(s) have one end joined to the expandable body and a second end joined to a moveable carrier and wherein movement of the moveable carrier allows an intermediate configuration to be selected; optionally wherein the moveable carrier can be locked against movement so as to hold the expandable body in an intermediate configuration.

9. A fixation device according to any preceding claim wherein the locking mechanism comprises a collet that can be opened or closed, preferably closed, to effect a locking action.

10. A fixation device according to any preceding claim wherein the expandable body takes the form of a stent and/or wherein the expandable body is formed from a nitinol material.

11. A fixation device according to any preceding claim wherein the clamp comprises at least one moveable jaw, the moveable jaw having thereon at least one gripping element to grip a native valve leaflet, optionally wherein the gripping element is moveable relative to the moveable jaw between a native valve leaflet gripping configuration of the moveable jaw where the gripping element is arranged to grip a native valve leaflet and a passive configuration of the moveable jaw where the gripping element is arranged so that it cannot grip a native valve leaflet.

12. A fixation device for implantation in an animal, comprising:
(a) a device body;
(b) at least one clamp on the device body, the clamp for engaging and holding tissue so as to hold the fixation device in place, such as the tissue of a native valve leaflet, the clamp comprising at least one moveable jaw, the moveable jaw being rotatable about a pivot axis; and
(c) a pinion mechanism including a pinion, and wherein the pinion mechanism is configured for rotating the moveable jaw about the pivot axis.

13. A fixation device according to Claim 12 wherein the pinion is on the moveable jaw, for example, integrally formed with the moveable jaw.

14. A fixation device according to claim 12 or 13 wherein the pinion has an axis of rotation that is coincident with the pivot axis of the moveable jaw.

15. A fixation device according to any preceding claim wherein the pinion mechanism is a rack and pinion mechanism that includes a rack; optionally wherein the rack is moveable in a reciprocating linear fashion, so that linear motion of the rack effects pivotal movement of the moveable jaw about the pivot axis.

16. A fixation device according to any of Claims 12 to 14 wherein the rack has teeth and the pinion has teeth and the two sets of teeth mesh with each other; optionally
wherein:
the rack has a rack body on which the teeth are formed and the teeth of the rack project above an upper surface of the rack body; and/or
the rack has a rack body in which the teeth are formed and the teeth of the rack are flush with an upper surface of the rack body.

17. A fixation device according to any of Claims 16 wherein the pinion mechanism further comprises a worm screw and the worm screw and the pinion are configured so that rotation of the worm screw rotates the pinion to effect rotation of the moveable jaw about the pivot axis.

18. A fixation device according to any of claims 12 to 17 wherein the pinion mechanism includes two pinions, on opposing sides of the moveable jaw.

19. A fixation device for implantation in an animal, comprising:
(a) a device body;
(b) at least one clamp on the device body, the clamp for engaging and holding tissue so as to hold the fixation device in place, such as the tissue of a native valve leaflet, the clamp comprising at least one moveable jaw, the moveable jaw being rotatable about a pivot axis; and
(c) a deployable shield that allows tissue to be caught by the clamp when the clamp is in a closed position but deploys to prevents tissue being caught by the clamp when the clamp is an least one open configuration.

20. A fixation device according to Claim 19 wherein the deployable shield is a flexible member that is pulled taut to form the deployable shield; and/or
wherein the moveable jaw has one or more tissue gripping elements thereon and the deployable shield prevents tissue being gripped by the gripping element(s) in an at least one open configuration; and/or
wherein the moveable jaw can open to an inverted position and the deployable shield is deployed in the inverted position.
